# EUROPEAN PATENT APPLICATION

(11) **EP 4 368 190 A1**
(43) Date of publication of application: **15.05.2024**
(21) Application number: 22837751.1
(22) Date of filing: 07.07.2022
(51) Int. Cl.: A61K 35/30, A01N 1/02, A61L 27/38, A61L 27/50, C12N 5/071

(54) **THERAPEUTIC COMPOSITION CONTAINING CORNEAL ENDOTHELIUM REPLACEMENT CELLS**

(30) Priority: 07.07.2021 US 202163219086 P; 17.03.2022 US 202263320846 P
(71) Applicant: Cellusion Inc., Tokyo 103-0024 (JP)
(72) Inventor: YOSHIZAKI, Shinji, Tokyo 103-0022 (JP); HATOU, Shin, Tokyo 103-0022 (JP); TSUKAMOTO, Masashi, Tokyo 103-0022 (JP)
(74) Representative: J A Kemp LLP
(86) International application number: PCT/JP2022/027002
(87) International publication number: WO 2023/282337

(57) **Abstract**

The present invention aims to provide a therapeutic composition, particularly a composition for transplantation therapy, which can be cryopreserved, does not cause damage to cells due to thawing, and can be administered to a patient as is without undergoing additional processes such as washing or centrifugation of cells after thawing. A therapeutic composition for transplantation containing a corneal endothelial substitute cell having a CD24-positive phenotype, phenolphthalein derivative, and a cryoprotective agent. Such therapeutic composition can be safely transplanted into the chamber of the eye, even though it contains a cryoprotective agent. Since it is CD24 positive, it can be expected to have an effect of avoiding phagocytosis.

## Description

### [Technical Field]

The present invention relates to therapeutic compositions, particularly, therapeutic compositions for transplantation, containing corneal endothelial substitute cells.

### [Background Art]

Corneal endothelial damage, such as decrease in corneal endothelial cells, impairs functions of corneal endothelial cell and causes edema in the corneal stroma. This decreases transparency of the cornea, and reduces the visual acuity. Such condition is called bullous keratopathy. In the meantime, it is known that human corneal endothelial cell once injured scarcely shows an ability to regenerate. When the corneal endothelial cells have decreased due to certain injury, an effective or sole treatment thereof is corneal transplantation. In fact, about half the number of applicable cases of corneal transplantation is for bullous keratopathy caused by corneal endothelial functional disorder.

At present, patients with corneal endothelium damage are treated by penetrating keratoplasty wherein the whole three-layer structure of corneal epithelium, corneal stroma and corneal endothelium is transplanted. While the penetrating keratoplasty is an established technique, the supply of cornea is short in Japan as the situation stands, and the rejection reaction poses a problem. To solve such problems, "part transplantation" involving transplantation of only the damaged tissue is becoming popular. Deep lamellar keratoplasty (DLKP) involving transplantation of only the epithelium and stroma of the donor while preserving corneal endothelium, corneal endothelium transplantation involving transplantation of only the part cornea including endothelium and the like are known. However, in the case of corneal endothelium transplantation, for example, the source of supply of the material for transplantation is still the corneal endothelium itself. Since the number of donor of cornea is limited, the problem of donor shortage cannot be overcome, like penetrating keratoplasty. Furthermore, since corneal endothelial cell is difficult to culture, preparation of cultured cells in a number sufficient for transplantation places a large burden in terms of time and cost.

In order to solve the problem of donor shortage, attempts are being made to create corneal endothelial cells or cells that have functions equivalent to those of corneal endothelial cells and can replace corneal endothelial cells.

For example, methods for inducing cells that can replace corneal endothelial cells from iPS cells or ES cells have been reported. Comprehensive review of the methods for inducing corneal endothelial cells (actually, since surface markers specific to corneal endothelial cells have not been established so far, cells with properties similar to those of corneal endothelial cells, so-called corneal endothelial like cells) from iPS cells or ES cells and cell culture methods is provided in Non Patent Literature 1. In addition, the present inventors have so far established a method for producing cells that have functions equivalent to those of corneal endothelial cells and can replace corneal endothelial cells (Patent Literatures 1 to 3), and named the corneal endothelial-like cell obtained by this method as "corneal endothelial substitute cell". Clinical research on iPS cell-derived corneal endothelial substitute cell for bullous keratopathy has also begun.

### [Citation List]

### [Patent Literature]

[Patent Literature 1]
   WO/2013/051722
[Patent Literature 2]
   WO/2016/093359
[Patent Literature 3]
   WO/2019/142833

### [Non Patent Literature]

[Non Patent Literature 1]
Hatou S, Shimmura S; Inflamm Regen. 2019; 39:19.

### [Summary of Invention]

### [Technical Problem]

In order to use cultured corneal endothelial cells or corneal endothelial substitute cells for actual clinical treatments, it is required to store produced cells stably until the time of treatment, and to transport them from the site of production to a hospital or clinic. However, cell viability and cell quality vary depending on the dosage form of cell recessed products and the temperature range during storage and transportation. It is therefore important to set a dosage form and a temperature range that achieve stable storage and stable transportation. In addition, it is desirable to have a dosage form that is as user-friendly as possible for treatment.

As a means to enable stable storage and stable transportation of cells for transplantation, a method of suspending cells, cell aggregates, or cell processed products thereof in a cryopreservation solution and cryopreserving them can be mentioned. The cryopreservation solution contains a cryoprotective agent such as dimethyl sulfoxide (DMSO) and the like. Generally, the presence of a cryoprotective agent affects cells and living organisms to which the cells are administered. Therefore, when using cells, cell aggregates, or cell recessed products for treatment, it is necessary to wash/remove the cryoprotective agent after thawing. Performing a step of washing/removing a cryoprotective agent is not desirable in terms of time and cost, and is highly damaging to the cells.

Therefore, the present invention aims to provide a therapeutic composition, particularly a composition for transplantation therapy, which can be cryopreserved, does not cause damage to cells due to thawing, and can be administered to a patient as is without undergoing additional processes such as washing or centrifugation of cells after thawing.

Corneal endothelial substitute cell is prepared by differentiating and inducing pluripotent stem cells, particularly induced pluripotent stem cells. However, it is difficult to induce all pluripotent stem cells into corneal endothelial substitute cell of interest, and stem cells to be the origin and undifferentiated cells that are in the process of differentiating and being induced into corneal endothelial substitute cell may remain. Such undifferentiated cells have proliferative activity, differentiate into cells other than corneal endothelial substitute cell, and may form teratomas when transplanted into a living body. Therefore, it is preferable that the cell population to be used in the present invention containing the corneal endothelial substitute cell has the lowest possible proportion of undifferentiated cells.

### [Solution to Problem]

In view of the above-mentioned problems, the present inventors have intensively studied a dosage form and a temperature range that achieve stable storage and stable transportation of cells, particularly cultured corneal endothelial cells or corneal endothelial substitute cell, and found that cells can be stably stored and transported by suspending them in a preservation solution and cryopreserving them and further that a therapeutic composition suitable for transplantation can be obtained by thawing and diluting the resulting cell-containing composition in the presence of a phenolphthalein derivative. The therapeutic composition does not require removal of the cryoprotective agent contained in the cryopreservation solution by washing or the like. Furthermore, the present inventors have confirmed that the cryopreserved cells maintain corneal endothelial function even after thawing and completed the present invention.

Furthermore, the present inventors have investigated the proportion of undifferentiated cells (hereinafter to be also referred to as undifferentiated cell residual rate) in a therapeutic pharmaceutical composition containing stem cell-derived corneal endothelial substitute cell, and found a pharmaceutically acceptable undifferentiated cell residual rate.

Therefore, the present invention provides the following.
[1] A therapeutic composition comprising a corneal endothelial substitute cell having a CD24-positive phenotype, and further a phenolphthalein derivative when desired.
[2] The composition of the above-mentioned [1], further comprising a cryoprotective agent.
[3] The composition of the above-mentioned [2], wherein the cryoprotective agent is dimethyl sulfoxide (DMSO).
[4] The composition of the above-mentioned [3], wherein a concentration of the DMSO in the composition is 1 to 10%.
[5] The composition of any of the above-mentioned [1] to [4], wherein the phenolphthalein derivative is selected from the group consisting of phenolsulfonphthalein (phenol red), phenolphthalein, bromophenol blue, isovaleryl phenolphthalein, acetylphenolphthalein, phenolphthalein dibutyrate, phenolphthalein diphosphate, phenolphthalein disulphate, phenolphthalein glucuronic acid, phenolphthalein mono-β-glucoside uronic acid, phenolphthalein mono-β-glucuronic acid, phenolphthalein mono-phosphate, and cresol red.
[6] The composition of any of the above-mentioned [1] to [4], wherein the phenolphthalein derivative is phenol red.
[7] The composition of the above-mentioned [6], wherein a concentration of the phenol red in the composition is 1 to 20 mg/L.
[8] The composition of any of the above-mentioned [1] to [7], wherein a concentration of the cell in the composition is 0.5 to 10×10⁶ cells/mL.
[9] The composition of any of the above-mentioned [1] to [8], which is in a dosage form of a spheroid cell suspension.
[10] The composition of the above-mentioned [9], wherein a cell population in the composition comprises spheroids having an area equivalent diameter of not less than 30 µm, and not less than 50% of the spheroids have an area equivalent diameter of 30 to 90 µm.
[11] The composition of any of the above-mentioned [1] to [10], wherein the corneal endothelial substitute cell is derived from a stem cell.
[12] The composition of the above-mentioned [11], wherein the stem cell is a pluripotent stem cell.
[13] The composition of the above-mentioned [12], wherein the pluripotent stem cell is derived from an iPS cell (including a cell imparted with an additional function by gene editing or gene transfer).
[14] The composition of the above-mentioned [13], wherein a B2M expression level is lower than that of a human corneal endothelial cell or a cultured endothelial cell.
[15] The composition of any of the above-mentioned [1] to [14], which is for transplantation.
[16] The composition of the above-mentioned [15], which is for transplantation into the anterior chamber.
[17] The composition of the above-mentioned [16], which is for treatment of a disease requiring corneal endothelial transplantation.
[18] The composition of the above-mentioned [17], wherein the disease is one kind selected from the group consisting of bullous keratopathy, circular conecornea, corneitis, chemical burn of cornea, corneal stromal dystrophy, corneal edema, and corneal leukoma.
[19] Use of a corneal endothelial substitute cell having a CD24-positive phenotype, for producing a therapeutic composition for a disease requiring corneal endothelial transplantation.
[20] The use of the above-mentioned [19], wherein the therapeutic composition comprises a phenolphthalein derivative when desired.
[21] The use of the above-mentioned [19] or [20], wherein the therapeutic composition further comprises a cryoprotective agent.
[22] The use of any of the above-mentioned [19] to [21], wherein the disease is one kind selected from the group consisting of bullous keratopathy, circular conecornea, corneitis, chemical burn of cornea, corneal stromal dystrophy, corneal edema, and corneal leukoma.
[23] A therapeutic composition for use in the treatment of a disease requiring corneal endothelial transplantation, comprising a corneal endothelial substitute cell having a CD24-positive phenotype.
[24] The composition of the above-mentioned [23], further comprising a phenolphthalein derivative when desired.
[25] The composition of the above-mentioned [23] or [24], further comprising a cryoprotective agent.
[26] The composition of any of the above-mentioned [23] to [25], wherein the disease is one kind selected from the group consisting of bullous keratopathy, circular conecornea, corneitis, chemical burn of cornea, corneal stromal dystrophy, corneal edema, and corneal leukoma.
[27] A method for treating a disease requiring corneal endothelial transplantation, comprising transplanting an effective amount of a therapeutic composition comprising a corneal endothelial substitute cell having a CD24-positive phenotype into an anterior chamber of a patient in need thereof.
[28] The method of the above-mentioned [27], wherein the therapeutic composition comprises a phenolphthalein derivative when desired.
[29] The method of the above-mentioned [27], wherein the therapeutic composition further comprises a cryoprotective agent.
[30] The method of any of the above-mentioned [27] to [29], wherein the disease is one kind selected from the group consisting of bullous keratopathy, circular conecornea, corneitis, chemical burn of cornea, corneal stromal dystrophy, corneal edema, and corneal leukoma.
[31] A method for producing a therapeutic composition, comprising adding a substrate for transplantation comprising a phenolphthalein derivative to a cell suspension or cell spheroid suspension of corneal endothelial substitute cells which is frozen at not more than -20°C, in the presence of a cryoprotective agent.
[32] The method of the above-mentioned [31], wherein the corneal endothelial substitute cell has a CD24-positive phenotype.
[33] A therapeutic composition comprising a corneal endothelial substitute cell derived from a stem cell, and having an undifferentiated cell residual rate of less than 1%.
[34] The composition of the above-mentioned [33], wherein the corneal endothelial substitute cell has a CD24-positive phenotype.
[35] The composition of the above-mentioned [33] or [34], which has an undifferentiated cell residual rate of not more than 0.1%.
[36] The composition of any of the above-mentioned [33] to [35], wherein the stem cell is a pluripotent stem cell.
[37] The composition of the above-mentioned [36], wherein the pluripotent stem cell is derived from an iPS cell (including a cell imparted with an additional function by gene editing or gene transfer).
[38] The composition of any of the above-mentioned [33] to [37], wherein a B2M expression level is lower than that of a human corneal endothelial cell or a cultured endothelial cell.
[39] The composition of any of the above-mentioned [33] to [38], wherein the undifferentiated cell residual rate is evaluated by expression profile of an undifferentiated cell marker.
[40] The composition of any of the above-mentioned [33] to [39], which is for transplantation.
[41] The composition of the above-mentioned [40], which is for transplantation into the anterior chamber.

### [Advantageous Effects of Invention]

According to the present invention, a therapeutic composition, particularly a composition for transplantation therapy, which can be cryopreserved, does not cause damage to cells due to thawing, and can be administered to a patient as is without undergoing additional processes such as washing or centrifugation of cells after thawing can be provided. The therapeutic composition of the present invention is useful for transplanting corneal endothelial substitute cell in bullous keratopathy, which accounts for about 50% of corneal transplantation applicable cases worldwide.

According to the present invention, transplantation without side effects becomes possible by confirming the undifferentiated cell residual rate in therapeutic compositions, particularly in compositions for transplantation.

### [Brief Description of Drawings]

[Fig. 1]

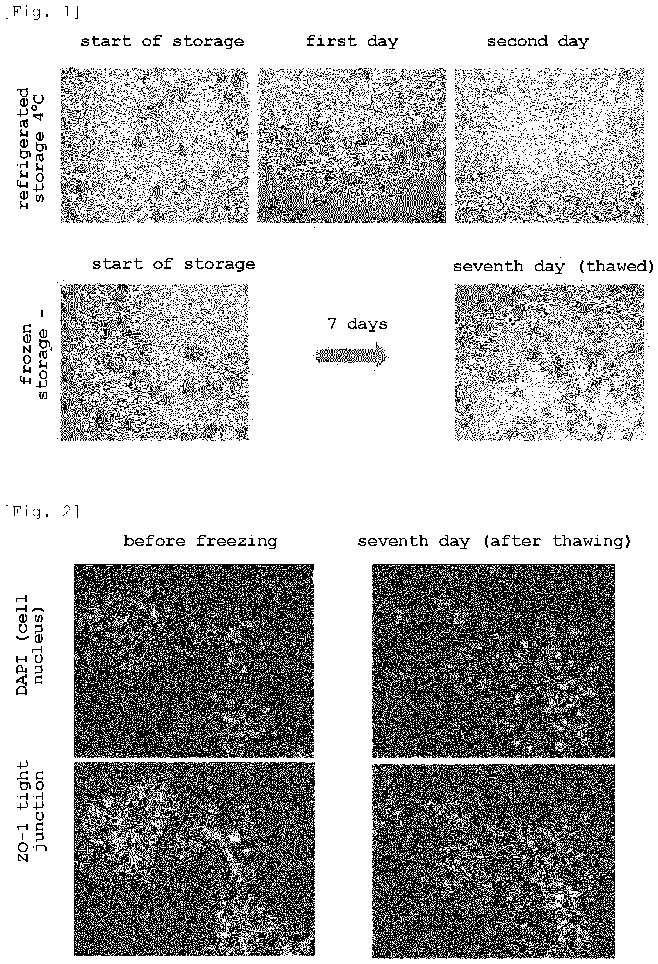

   Fig. 1 is a diagram showing the comparison results of the state of corneal endothelial substitute cell between when they are stored refrigerated and when they are stored frozen. It was found that frozen storage can maintain the cell aggregates for a long period of time.
[Fig. 2]
   Fig. 2 shows formation of tight junction at the boundary of corneal endothelial substitute cell after thawing in the therapeutic composition of the present invention. Expression of ZO-1 was found at the cell boundary.
[Fig. 3]

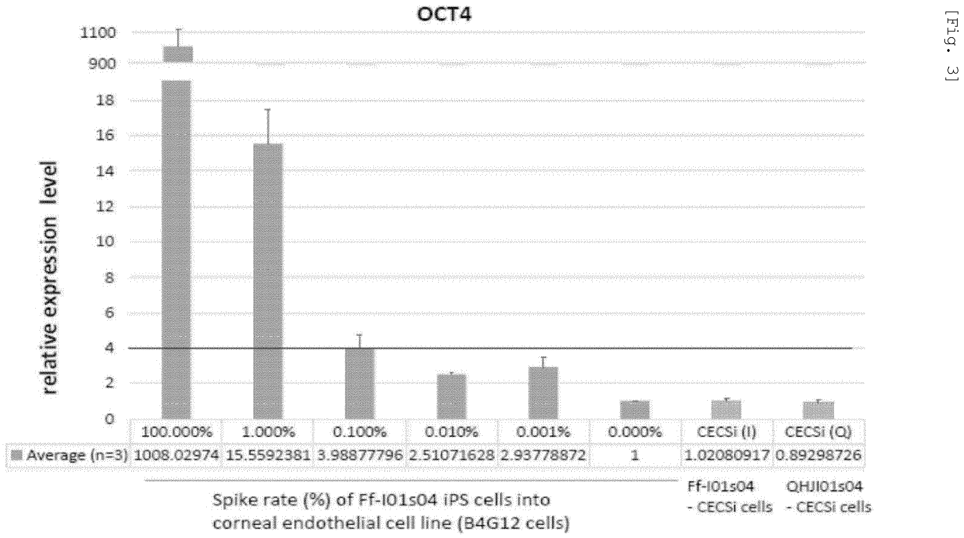

   Fig. 3 is a diagram showing the examination results of the undifferentiated cell residual rate.
[Fig. 4]

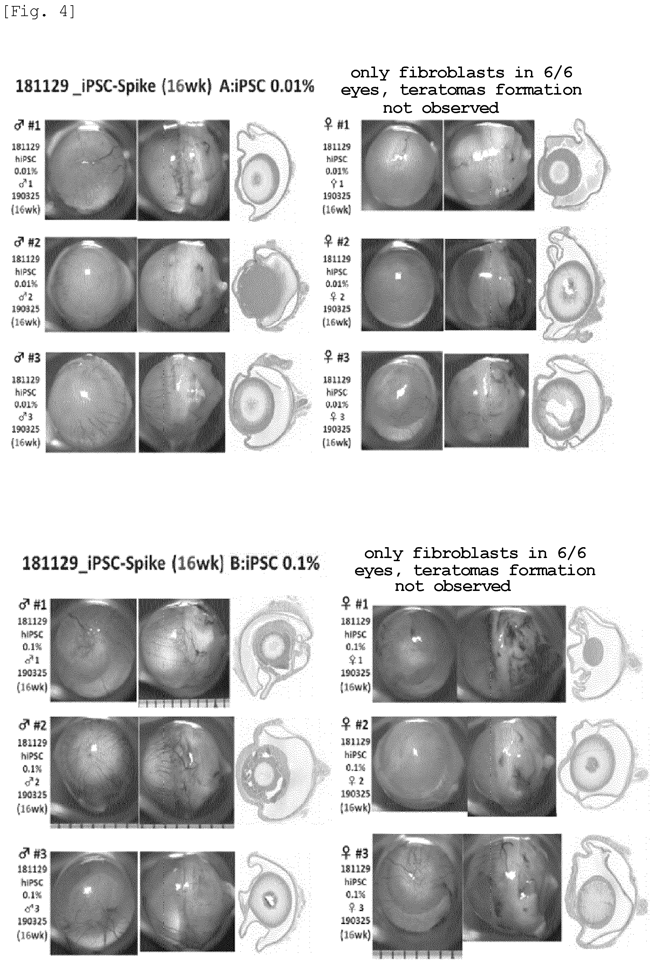

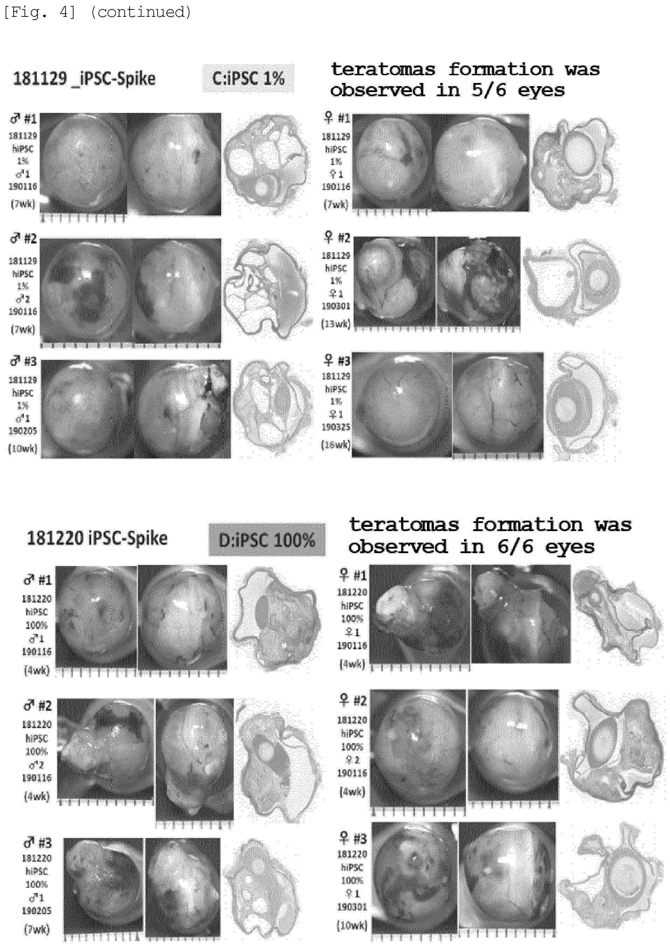

   Fig. 4 is a diagram examining the influence of undifferentiated cell spike rate on teratoma formation. Teratoma formation requires a spike rate of not less than 1%, and teratoma formation was not confirmed at spike rates of 0.1% and 0.01%.
[Fig. 5]

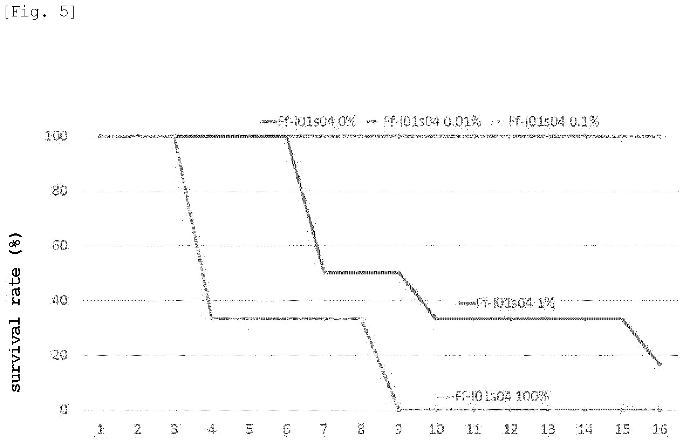

   Fig. 5 is a graph showing a survival curve (Kaplan-Meier curve) from an undifferentiated iPS cell spike test.
[Fig. 6]

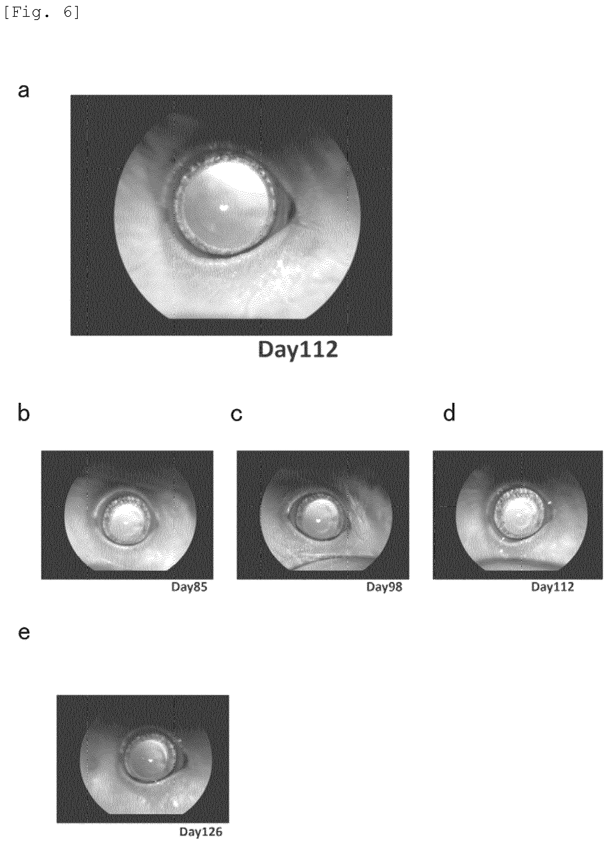

   Fig. 6 is a diagram showing the results of transplanting corneal endothelial substitute cell with an undifferentiated cell residual rate of not more than 0.1% into the anterior chamber of a nude rat and observing the progress. The progress was followed for up to 26 weeks, but no tumorigenicity was observed.
[Fig. 7]

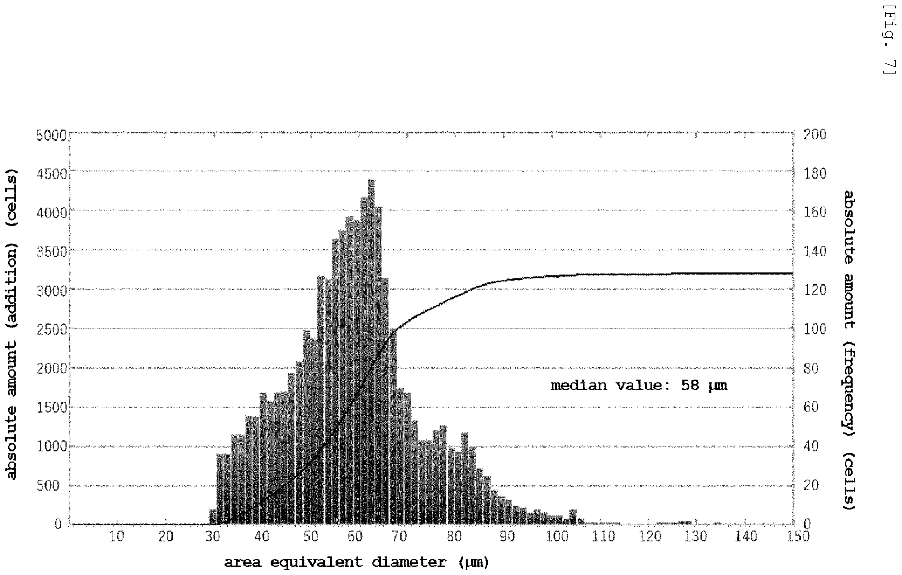

   Fig. 7 is a graph showing the results of verifying the size of spheroidized corneal endothelial substitute cell.
[Fig. 8]

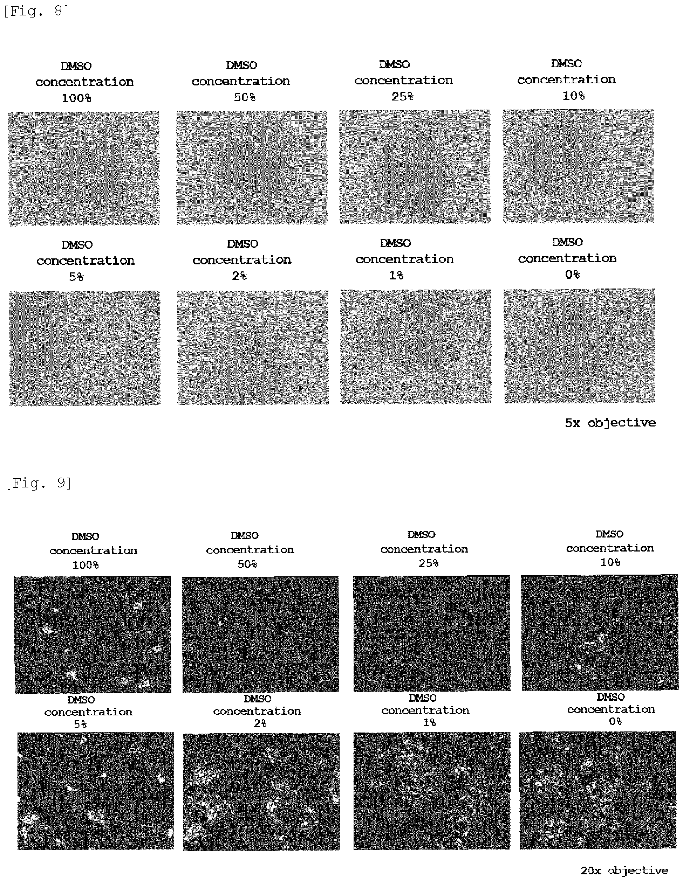

   Fig. 8 shows microscopic observation images of CECSi cells at different concentrations of cryoprotective agent.
[Fig. 9]
   Fig. 9 shows stained images of anti-ZO-1 antibody of CECSi cells at different concentrations of cryoprotective agent.
[Fig. 10]

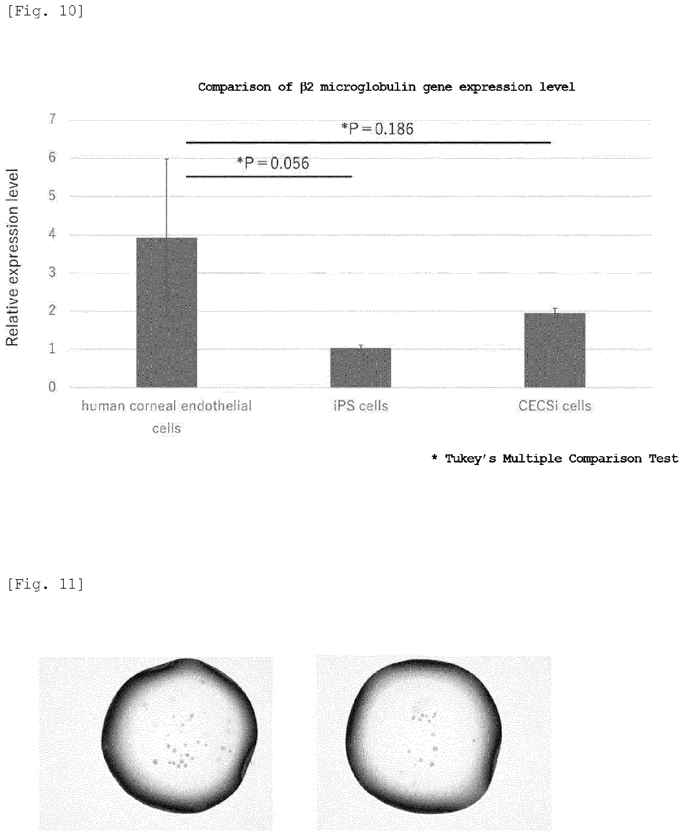

   Fig. 10 is a graph showing the comparison results of the expression levels of beta-2 microglobulin (B2M) in human corneal endothelial cells, iPS cells (Ff-I01s04), and CECSi cells.
[Fig. 11]
   Fig. 11 is a diagram showing micrographs of a CECSi cell spheroid suspension (left: without phenol red, right: with phenol red).

### [Description of Embodiments]

The present invention is explained in the following. Unless particularly indicated, the terms used in the present specification have the meanings generally used in the pertinent field.

### 1. Therapeutic composition

The present invention provides a therapeutic composition, particularly, a therapeutic composition for transplantation, containing a cultured corneal endothelial cell or a corneal endothelial substitute cell, and a phenolphthalein derivative. Preferably, it is a therapeutic composition, particularly, a therapeutic composition for transplantation, containing a corneal endothelial substitute cell and a phenolphthalein derivative. The present invention provides a therapeutic composition containing a corneal endothelial substitute cell derived from a stem cell, which is a composition characteristically having an undifferentiated cell residual rate of less than 1%. In the present specification, these are sometimes to be comprehensively abbreviated as the therapeutic composition of the present invention. The therapeutic composition is preferably used for intraocular transplantation of corneal endothelial substitute cells or cultured corneal endothelial cells, particularly transplantation into the anterior chamber.

### (Cell)

In the therapeutic composition of the present invention, a cultured corneal endothelial cell or a corneal endothelial substitute cell, preferably a corneal endothelial substitute cell, more preferably an iPS cell-derived corneal endothelial substitute cell, is used.

While the cultured corneal endothelial cells may be primary cultured cells or established line of cells, established line of cells are preferably used since primary culture of corneal endothelial cells is difficult.

In the present invention, the "corneal endothelial substitute cell" is a cell induced from a stem cell such as iPS cell or ES cell, or such cell imparted with additional functions through gene editing or gene transfer, and the like, and can treat a corneal endothelium functional disorder and replace corneal endothelial cells. That is, the corneal endothelial substitute cell has physiological functions equivalent to those of the corneal endothelial cell.

The "stem cell" means a cell capable of being cultured in vitro, and capable of differentiating into plural lines of cells constituting the body. Specific examples include embryonic stem cell (ES cell), fetal primordial germ cell-derived pluripotent stem cell (EG cell), testis-derived pluripotent stem cell (GS cell), somatic cell-derived artificial pluripotent stem cell (induced pluripotent stem cells; iPS cell), and human somatic stem cell (tissue stem cell). As the iPS cell, an iPS cell derived from any warm-blooded animal, preferably a mammal, can be used. Examples of the mammal include mouse, rat, guinea pig, hamster, rabbit, cat, dog, sheep, swine, bovine, horse, goat, monkey, human and the like. A cell derived from human can be preferably used.

Specific examples of the iPS cell include cells obtained by introducing plural genes into a somatic cell such as skin cell and the like, which have acquired multipotency same as that of ES cell. Examples thereof include iPS cell obtained by introducing Oct3/4 gene, Klf4 gene, C-Myc gene and Sox2 gene, iPS cell obtained by introducing Oct3/4 gene, Klf4 gene and Sox2 gene (Nat Biotechnol 2008; 26: 101-106) and the like. Furthermore, the production method of iPS cell has been intensively improved technically, for example, a method involving further reducing transgenes (Nature. 2008 Jul 31; 454(7204): 646-50), a method utilizing a low-molecular-weight compound (Cell Stem Cell. 2009 Jan 9; 4(1): 16-9, Cell Stem Cell. 2009 Nov 6; 5(5): 491-503), a method utilizing a transcription factor protein instead of the gene (Cell Stem Cell. 2009 May 8; 4(5): 381-4) and the like. Since the basic property of the iPS cells produced, that is, having multipotency, is equivalent irrespective of the production methods, all of them can be the origin of the corneal endothelial substitute cell used in the present invention.

Specifically, as the iPS cell, 201B7, 201B7-Ff, 253G1, 253G4, 1201C1, 1205D1, 1210B2, 836B3, FF-I14s03, FF-I01s04, MH09s01, Ff-XT18s02, Ff-WIs03, Ff-WJs513, Ff-CLs14, Ff-KVs09, QHJI14s03, QHJI01s04, RWMH09s01, DRXT18s02, RJWIs03, YZWJs513, ILCLs14, GLKVs09, Ff-XT28s05-ABo_To, Ff-I01s04-ABII-KO, Ff-I14s04-ABII-KO (all iPS Academia Japan, Inc., or CiRA Foundation), Tic (JCRB1331 strain), Dotcom (JCRB1327 strain), Squeaky (JCRB1329 strain), and Toe (JCRB1338 strain), Lollipop (JCRB1336 strain) (National Center for Child Health and Development, National Institutes of Biomedical Innovation, Department of Intractable Diseases and Disease Resources, JCRB Cell Bank), UTA-1 strain and UTA-1-SF-2-2 strain (all The University of Tokyo), 21526, 21528, 21530, 21531, 31536, 31538 strains (all FUJIFILM Cellular Dynamics, Inc.), and the like can be used.

The corneal endothelial substitute cell contained in the therapeutic composition of the present invention is, for example, a corneal endothelial-like cell developed by the present inventors (Patent Literatures 1 to 3), and can be produced and prepared by the methods described in Patent Literatures 1 to 3. Preferably, it is a corneal endothelial substitute cell derived from an iPS cell (including cells imparted with additional functions through gene editing or gene introduction) (Corneal Endothelial Cell substitute from iPS Cells; CECSi cell), which has properties and functions similar to those of corneal endothelial cells and has enhanced gene expression level of NR3C2 (nuclear receptor subfamily 3, group C, member 2) (Patent Literature 3).

The corneal endothelial cell-like properties and functions that the corneal endothelial substitute cell has specifically include the following characteristics (i) to (iv). Corneal endothelial substitute cell has at least one, preferably two, more preferably three, and even more preferably all four, of these characteristics.
(i) Intercellular adhesion is composed of N-Cadherin.
(ii) Tight junction is formed between cells.
(iii) Na,K-ATPase α1 subunit is expressed on the cell membrane.
(iv) Expression of transcription factor PITX2 is observed in the cell nucleus.

Whether or not intercellular adhesion is composed of N-Cadherin can be confirmed by immunostaining for N-Cadherin.

Whether or not tight junction is formed between cells can be confirmed by observing the presence of ZO-1, which is a protein constituting tight junctions, by immunostaining for ZO-1. It can also be confirmed by directly observing the structure using an electron microscope.

Whether or not Na,K-ATPase α1 subunit (ATP1A1) is expressed on the cell membrane can be confirmed by co-staining of ZO-1 and Na,K-ATPase α1 subunit by immunostaining.

Whether or not the transcription factor PITX2 is expressed in the cell nucleus can be confirmed by immunostaining for PITX2.

In one embodiment of the present invention, the corneal endothelial substitute cell contained in the therapeutic composition of the present invention is characterized by having a CD24-positive phenotype. CD24 is a highly glycosylated, small mucin-like glycosylphosphatidyl-inositol (GPI)-binding cell surface protein. CD24 is highly expressed on hematopoietic cells such as B cells, T cells, neutrophils, eosinophils, dendritic cells, and macrophages, as well as non-hematopoietic cells such as neurons, ganglion cells, epithelial cells, keratinocytes, muscle cells, pancreatic cells, and epithelial stem cells. In recent years, it has been reported that CD24 has the function of avoiding phagocytosis by macrophages. Therefore, the therapeutic composition of the present invention containing corneal endothelial substitute cell having a CD24-positive phenotype can be expected to reduce rejection reactions in transplantation. On the other hand, it has been reported that human corneal endothelial cells are CD24 negative (JP-A-2019-510509, JP-A-2020-073602).

The presence or absence of a CD24-positive phenotype can be confirmed by immunostaining for the CD24 antigen expressed on the cell surface.

Each immunostaining is generally performed in the art, and the reagents, equipment, and the like to be used are commercially available or can be prepared according to previous reports.

The cultured corneal endothelial cell or corneal endothelial substitute cell in the therapeutic composition is preferably spheroid. In the present specification, the "spheroid" is a cell mass formed by aggregation of several dozen to several hundred cells, and generally has a spherical shape. As used herein, "spherical" includes a complete sphere, as well as substantially spherical shapes such as an egg shape and a rugby ball shape.

The spheroid has a diameter or area equivalent diameter in the range of preferably 10 to 200 µm, more preferably in the range of 30 to 150 um, particularly preferably in the range of 30 to 100 µm, even more preferably in the range of 30 to 90 um, and even further preferably in the range of 40 to 80 um. When the spheroid is too large, engraftment becomes uneven and not uniform, and when the spheroid is too small, engraftment becomes difficult and the engraftment efficiency decreases. When the size of the spheroid is within this range, cells are efficiently engrafted at the transplantation site. The "area equivalent diameter" refers to the diameter of a circle that is equal to the projected area of a particle (in the present case, a spheroid), and is also referred to as the Heywood diameter or equivalent circle diameter. That is, it is the diameter of a circle having an area equal to the projected area of a substantially globular spheroid.

The therapeutic composition of the present invention is provided as a cell suspension of cultured corneal endothelial cells or corneal endothelial substitute cells, preferably a dosage form of a spheroid suspension.

The concentration of the cultured corneal endothelial cell or corneal endothelial substitute cell in the therapeutic composition is generally 0.5 to 10×10⁶ cells/mL, preferably 1.0 to 5×10⁶ cells/mL. Too high or too low a cell concentration reduces the engraftment efficiency at the transplantation site.

When the dosage form of the therapeutic composition of the present invention is a suspension, cell concentration is determined by sampling a part of the cell suspension and counting the cells in the sample; and when the dosage form is a spheroid suspension, cell concentration is determined by sampling a part of the spheroid suspension, dispersing the cells into single cells by an enzyme treatment of the spheroid in the sample, counting the cells in the sample and using the results thereof.

When the dosage form is a spheroid suspension, the cell population contained in the therapeutic composition of the present invention contains spheroids with an area equivalent diameter of not less than 30 um. Not less than 50%, preferably not less than 70%, more preferably not less than 80%, further preferably not less than 90%, of the spheroids contained in the spheroid suspension are within the range of median value ±30 µm of the diameter or area equivalent diameter. When the variation in the diameter or area equivalent diameter of one spheroid is less, the engraftment surface can be covered more efficiently and more uniformly. In an embodiment, not less than 50%, preferably not less than 70%, more preferably not less than 80%, further preferably not less than 90%, of spheroid has a diameter or area equivalent diameter of 30 to 90 µm.

The therapeutic composition containing a corneal endothelial substitute cell of the present invention may contain stem cells to be the origin, such as iPS cells and cells that are in the process of differentiating and being induced into corneal endothelial substitute cell (hereinafter to be also referred to as undifferentiated cells). The greater the proportion of undifferentiated cells is, the more likely side effects such as teratoma formation and the like occur when transplanted into a living body. The therapeutic composition containing a corneal endothelial substitute cell of the present invention preferably has an undifferentiated cell residual rate of less than 1%, more preferably not more than 0.1%, still more preferably not more than 0.01%. By suppressing the undifferentiated cell residual rate to less than 1%, it is possible to suppress teratoma formation and tumor formation when transplanted into a living body.

As used herein, the "undifferentiated cell residual rate" means the proportion of undifferentiated cells in the total cells contained in the therapeutic composition of the present invention. When the undifferentiated cell rate is 0%, it means that all of the cells in the therapeutic composition of the present invention are corneal endothelial substitute cells, and when the undifferentiated cell rate is 100%, it means that all of the cells in the therapeutic composition of the present invention are undifferentiated cells. When the undifferentiated cell residual rate is less than 1%, not more than 0.1%, or not more than 0.01%, it means that less than 1%, not more than 0.1%, or not more than 0.01%, respectively, of the total cells in the therapeutic composition of the present invention is undifferentiated cells. When the total number of cells in the therapeutic composition of the present invention is 1×10⁴ cells and the undifferentiated cell residual rate is less than 1%, not more than 0.1%, or not more than 0.01%, less than 100, not more than 10, not more than 1, respectively, cell(s) may be undifferentiated cell(s).

The measurement of the undifferentiated cell residual rate is not particularly limited and can be performed by, for example, evaluating the expression profile of a cell marker specific to undifferentiated cells (hereinafter also to be referred to as "undifferentiated cell marker"). In the present specification, the undifferentiated cell marker is a gene or protein that is specifically expressed in the stem cells from which it is derived, and transcription factors NANOG, OCT4, and SOX2, surface antigen SSEA4, TRA-1-60, TRA-1-81, and the like can be mentioned. It is preferably performed by evaluating OCT4 expression (Stem Cell Research 55 (2021) 102497). OCT4 is a factor used as a marker for pluripotent cells. ES cells and iPS cells express this gene but non-pluripotent cells such as skin cells and heart cells do not express OCT4. Specifically, it can be evaluated by the method described in the Examples. Quantitative PCR of OCT4 is performed on the composition, and when the OCT4 expression level is not more than 4.0 times that in B4G12 cells, which is an established line of human corneal endothelial cells, it corresponds to "undifferentiated cell residual rate of not more than 0.1%". By similarly confirming in advance the correlation between the expression level and the undifferentiated cell residual rate for other undifferentiated cell markers, it can be determined whether the therapeutic composition containing the corneal endothelial substitute cell of the present invention is suitable for transplantation.

### (Phenolphthalein derivative)

The therapeutic composition of the present invention is characterized in that it contains a phenolphthalein derivative in addition to the above-mentioned cells. The phenolphthalein derivative is not particularly limited as long as it has the basic skeleton represented by the following formula to achieve the desired effect. 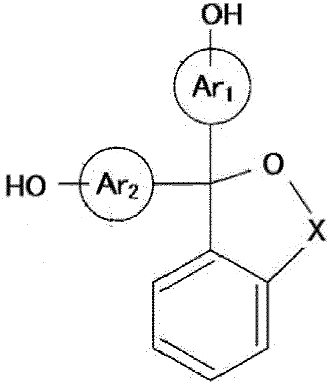 wherein -X- is -SO₂- or -CO-, and ring Ar₁ and ring Ar₂ are the same or different and each is an aromatic ring having 6 or 12 carbon atoms (e.g., benzene, naphthalene) optionally having 1 to 3 substituents (e.g., alkyl groups optionally having a branched chain having 1 to 3 carbon atoms such as methyl, ethyl, propyl, isopropyl and the like; halogen atoms such as bromine atom, chlorine atom, and the like; acyl groups such as acetyl, isovaleryl, and the like).

Preferably, -X- is -SO₂-. Ring Ar₁ and ring Ar₂ are the same or different and each is benzene optionally having 1 to 3, preferably 1 or 2 substituents (e.g., methyl, isopropyl, bromine atom).

The phenolphthalein derivative may be a salt thereof or an ester thereof. Alternatively, it may be an acid adduct.

Examples of the phenolphthalein derivative include phenolsulfonphthalein(phenol red), phenolphthalein, bromophenol blue, isovaleryl phenolphthalein, acetylphenolphthalein, phenolphthalein dibutyrate, phenolphthalein diphosphate, phenolphthalein disulphate, phenolphthalein glucuronic acid, phenolphthalein mono-β-glucosiduronic acid, phenolphthalein mono-β-glucuronic acid, phenolphthalein mono-phosphate, and cresol red. The phenolphthalein derivative can be produced by a method known per se. It is known that phenolphthalein derivatives can be synthesized from phthalic acid and various corresponding phenols, and when the phenols used is phenol, phenolphthalein can be synthesized. Also, various phenolphthalein derivatives are commercially available. From the viewpoint of convenience and stability of quality, it is preferable to use commercially available products. Preferably, the phenolphthalein derivative is phenol red (also to be referred to as phenolsulfonephthalein) having the following structure. Phenol red is generally included in a medium as a pH indicator, but is an optional addition component from the viewpoint of cell culture, and phenol red-free medium is also commercially available. The concentration of phenol red contained in the therapeutic composition of the present invention is generally 1 to 20 mg/L, preferably 1 to 15 mg/L, more preferably 5 to 10 mg/L. The concentration of phenolphthalein derivative other than phenol red is also generally 1 to 20 mg/L, preferably 1 to 15 mg/L, more preferably 5 to 10 mg/L.

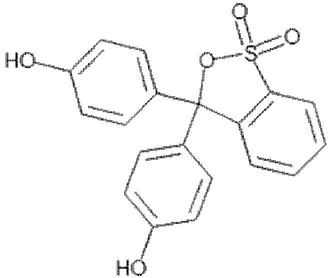

The "desired effect" of the phenolphthalein derivative is the effect of promoting engraftment of cells after transplantation.

Examples of the subject of administration of the therapeutic composition of the present invention include mammals such as mouse, rat, hamster, guinea pig, rabbit, cat, dog, bovine, horse, sheep, monkey, human and the like, and preferred is human.

The dose of the therapeutic composition of the present invention varies depending on the body weight, age, symptom and the like of the subject of administration. For example, 50 to 200 µl per eye is administered by injection into the anterior chamber of human weighing 25 to 300 Kg, age 16 to 100 years, with bullous keratopathy as a symptom. Injection of the therapeutic composition containing a cryoprotective agent, a substrate for transplantation, and cells or cell spheroids according to the present invention into the anterior chamber is safe and does not cause side effects such as an increase in intraocular pressure or significant inflammation.

The therapeutic composition of the present invention can be preferably used to treat diseases requiring corneal endothelium transplantation. Such diseases include, but are not limited to, bullous keratopathy, circular conecornea, corneitis, chemical burns of the cornea, corneal stromal dystrophy, corneal edema, corneal leukoma, and the like.

The present invention can also provide a method for treating a disease requiring corneal endothelium transplantation, which includes transplanting an effective amount of the therapeutic composition into the anterior chamber of a patient in need thereof. Here, the applicable diseases and dosages are those mentioned above.

### 2. Production method of the therapeutic composition

The therapeutic composition of the present invention can be prepared by, for example, spheroidizing the above-mentioned cultured corneal endothelial cells or corneal endothelial substitute cells, preferably corneal endothelial substitute cells, more preferably CECSi cells, suspending and freezing them in a cryopreservation solution, storing them for a certain period of time, and then thawing and diluting them in the presence of a phenolphthalein derivative.

As a method for spheroidizing cells, hanging drop method, rotating culture method, and culture in a low-adherence culture container are known. The method of culture in a low-adherence culture container is preferably used since the operation is easy. When adherent cells grown in a general culture container are cultured in a low-adherence container, the cells adhere to each other and form spheroids due to the properties of the cells themselves. For example, cells can be made into spheroids by suspension culture in a container such as EZSphere (registered trademark, AGC TECHNO GLASS CO., LTD.), WellBag (TOYO SEIKAN CO. LTD.), or the like. A spheroid-forming medium is preferably used for spheroidization. Examples of the spheroid-forming medium include, but are not limited to, the media described in the Examples. By not containing a phenolphthalein derivative in the spheroid-forming medium, more spheroids can be easily formed.

In the present invention, the method of freezing and the method of preserving cells or spheroidized cells are not particularly limited, and methods generally practiced for cryopreservation of cells can be employed. The cells are frozen and preserved at preferably -70°C or lower, more preferably -80°C or lower. The preservation period is not particularly limited, and is generally several weeks to half a year, preferably two weeks to six months, more preferably four months, during which the cells can be preserved without impairing the survival rate after thawing.

Cryopreservation of cells refers to cryopreserving cells for the purpose of maintaining cells for a long period of time without passaging. It is known that when cells are frozen as they are, ice crystals are generated due to the water contained within the cells, and the grown ice crystals destroy the cell membrane, causing so-called frost damage. Therefore, the cryopreservation solution used during cryopreservation contains a cryoprotective agent for the purpose of preventing frost damage. Therefore, the therapeutic composition of the present invention may contain a cryoprotective agent in addition to the cells and the phenolphthalein derivative.

The cryoprotective agent is not particularly limited as long as the purpose of preventing frost damage is achieved. Examples thereof include cell-permeable cryoprotective agent, cell-impermeable cryoprotective agent, and the like. Examples thereof include dimethyl sulfoxide (DMSO), ethylene glycol, carboxylated polylysine, glycerol, propylene glycol, sericin, propanediol, dextran, polyvinylpyrrolidone, poly(vinyl alcohol), hydroxyethylstarch, chondroitin sulfate, polyethylene glycol, formamide, acetamide, adonitol, perseitol, raffinose, lactose, trehalose, sucrose, mannitol, and the like. Cryoprotective agents may be used alone or in combination of two or more. Preferred is DMSO which is widely used.

The cryopreservation solution may include a medium to dissolve and/or dilute the cryoprotective agent and maintain cell survival. The medium is not particularly limited as long as it has the above-mentioned functions, and physiological saline, various physiological buffers (e.g., PBS, HBSS, etc.), and those based on various basal media for cell culture may be used. The basal medium is not particularly limited as long as it can be used for culturing animal cells, and includes, for example, MEM medium, BME medium, BGJb medium, CMRL 1066 medium, Glasgow MEM medium, Improved MEM Zinc Option medium, IMDM medium, Medium 199 medium, Eagle MEM medium, αMEM medium, DMEM medium, ham medium (e.g., F10, F12), RPMI 1640 medium, Fischer's medium, and a mixed medium thereof. These media are commercially available. Furthermore, these media can be a serum-containing medium or serum-free medium, preferably a serum-free medium. When the medium to be used in the present invention is a serum-containing medium, mammalian sera such as bovine serum, fetal bovine serum and the like can be used. The concentration of the serum in a medium is 0.1 - 20% (v/v), preferably 1 - 10% (v/v).

The concentration of the cryoprotective agent added to the cryopreservation solution or the concentration of the cryoprotective agent in the cryopreservation solution is not particularly limited as long as it does not excessively deteriorate the quality of cells during freezing and thawing operations. Typically, it is, for example, about 1% to about 60% (v/v), about 2% to about 50% (v/v), about 5% to about 40% (v/v), about 5% to about 20% (v/v), about 10% to about 20% (v/v), or the like of the entire cryopreservation solution. When the cryoprotective agent is DMSO, it is contained, for example, at 10-20% in the cryopreservation solution.

Commercially available cryopreservation solutions that contain a cryoprotective agent in advance (e.g., CELLBANKER (registered trademark), BAMBANKER (registered trademark)) can also be preferably used.

The thus-frozen composition containing cultured corneal endothelial cells or corneal endothelial substitute cells (preferably corneal endothelial substitute cells, more preferably CECSi cells) and a cryopreservation solution containing a cryoprotective agent is referred to as the "cell-containing composition" of the present invention. The therapeutic composition of the present invention can be prepared by thawing and diluting the cell-containing composition in the presence of a phenolphthalein derivative.

A step of thawing and diluting the cryopreserved cell-containing composition in the presence of a phenolphthalein derivative can be specifically performed by adding a substrate for transplantation to a cryopreserved cell-containing composition. Generally, 1 to 9 parts by weight (2- to 10-fold dilution), more preferably 4 parts by weight (5-fold dilution), of the substrate for transplantation is added to 1 part by weight of the cell-containing composition. The concentration of the cryoprotective agent in the composition after addition of the substrate for transplantation is preferably not more than 2%, more preferably not more than 1%.

The phenolphthalein derivative can be included only in the substrate for transplantation or in both the cryopreservation solution and the substrate for transplantation. It is preferably included only in the substrate for transplantation.

The concentration of the phenolphthalein derivative added to the cryopreservation solution and/or the substrate for transplantation, or the concentration of the phenolphthalein derivative in the cryopreservation solution and/or the substrate for transplantation, is not particular limited as long as influence on the cells or living organisms by the cryoprotective agent can be eliminated. The final concentration, i.e., the concentration in the therapeutic composition, can be adjusted to 1 to 20 mg/L, preferably 1 to 15 mg/L, more preferably 5 to 10 mg/L. For example, when the phenolphthalein derivative is phenol red and the concentration in the substrate for transplantation is 6 to 12 mg/L, the concentration of phenol red in the therapeutic composition will be 5-10 mg/L by diluting the cell-containing composition 5 times with the substrate for transplantation.

A therapeutic composition is prepared by diluting the cell-containing composition 2- to 10-fold, preferably 5-fold, with a substrate for transplantation. Thus, the cryoprotective agent may be contained at 0.1 to 30% (v/v), 0.2 to 25% (v/v), 0.5 to 20% (v/v), 0.5 to 10% (v/v), 1 to 10% (v/v), or the like, or 0.2 to 12% (v/v), 0.4 to 10% (v/v), 1 to 8% (v/v), 1 to 4% (v/v), 2 to 4% (v/v), or the like, in the therapeutic composition during cryopreservation. When the cryoprotective agent is DMSO, the concentration is, for example, 10 to 20% (v/v) in the cryopreservation solution, and not more than 5% (v/v), preferably not more than 2% (v/v), in therapeutic compositions at the time of transplantation.

While the substrate for transplantation is not particularly limited as long as it is a solution suitable for intraocular administration, particularly administration into the anterior chamber, a nonirritating isotonized culture medium, saline, buffer and the like are used. Preferably, a culture medium similar to one used for culturing the cultured corneal endothelial cell or corneal endothelial substitute cell is used. The substrate for transplantation can contain various components as necessary besides phenolphthalein derivative. While the component is not particularly limited as long as it is useful for differentiation of the cultured corneal endothelial cell or corneal endothelial substitute cell, that has been administered (transplanted) into the anterior chamber, into corneal endothelial cell layer without falling off, it is preferable to add insulin or insulin-like growth factor. Also, retinoic acid, EGF and bFGF are also preferable components to be added.

### [Example]

The present invention is described in detail in the following by referring to Examples, which are not to be construed as limitative. Unless particularly specified, the reagents and materials to be used are commercially available. Abbreviations used in the present specification are the same as those generally used in the art, unless otherwise specified.

### Example 1: Verification of preservation conditions for spheroidized corneal endothelial substitute cell

By a method similar to that in Example 4 of Patent Literature 3, CECSi cells were induced from iPS cells, and on the 14th day, the cells were collected using Accutase (Nacalai Tesque #12679-54), suspended in cryopreservation solution BAMBANKER (registered trade mark) hRM (NIPPON Genetics Co, Ltd. #CS-07-001; containing 10% DMSO) at a cell concentration of 1×10⁷ cells/mL, and cryopreserved in a deep freezer at -80°C. After 7 months, the cryopreserved cells were thawed and cultured using a spheroid-forming bag (TOYO SEIKAN CO. LTD., WellBag) to spheroidize CECSi cells.

Spheroidized CECSi cells were collected, suspended in cryopreservation solution BAMBANKER (registered trademark) hRM (NIPPON Genetics Co, Ltd. #CS-07-001; containing 10% DMSO) at a cell concentration of 6×10⁶ cells/mL, and cryopreserved in a deep freezer at -80°C (frozen storage). Seven days later, the cryopreserved cells were thawed by adding 4-fold volume of phenol red-containing substrate for transplantation (containing 8.1 mg/L phenol red), and the shape of the spheroid was confirmed. As the substrate for transplantation, the CECSi cell induction (+T) medium used in Example 4 of Patent Literature 3 was used. Similarly, CECSi cells induced from iPS cells were spheroidized, stored refrigerated at 4°C (refrigerated storage), and the shape of the spheroid was confirmed on the first and second days after storage. The results are shown in Fig. 1.

In refrigerated storage, most spheroids were already dispersed on the second day after storage, but it was confirmed that most of the spheroids maintained their shape after thawing even after 7 days of frozen storage for the frozen cells.

From these results, it was confirmed that spheroidized corneal endothelial substitute cells can be preserved for a long period of time by cryopreservation.

Furthermore, the spheroidized corneal endothelial substitute cells after thawing were seeded into a culture container, and it was examined whether the corneal endothelial substitute cells maintained corneal endothelium-like functions.

Barrier function, which is one of the important functions of corneal endothelial substitute cells, was evaluated using the ability to form tight junction as an index.

The spheroidized corneal endothelial substitute cells before cryopreservation or after 7 days of cryopreservation were seeded in a 48-well plate, and the cells were allowed to adhere to the plate. After 24 hr, the medium was removed from the cells on the wells and the cells were fixed with ice-cooled 4% para-formaldehyde. After washing the wells twice with PBS, blocking solution (PBST containing 10% Normal Donkey Serum (10% NDS/PBST)) was added and blocking was performed for 30 min. Thereafter, a primary antibody reaction was performed at room temperature for 1 hr using the following antibody amounts. Rabbit anti human ZO-1; Invitrogen, 40-2200, 1:500, 0.4 µL/well blocking solution (10% NDS/PBST), 0.20 mL/well

Then, the well was washed twice with PBS, and a secondary antibody reaction was performed at room temperature for 1 hr using the following antibody amounts.
Donkey anti-rabbit Cy-3, Jackson, 711-165-152, 1:200, 1.00 µL/well
DAPI, 1:1000, 0.20 µL/well
blocking solution (10% NDS/PBST), 0.20 mL/well

Then, after washing twice with PBS, a mounting agent was added dropwise and observed with a fluorescence microscope.

The results are shown in Fig. 2. In the spheroids after cryopreservation, ZO-1 expression was confirmed at the cell boundaries, like before freezing. From the above results, it was confirmed that the cryopreserved corneal endothelial substitute cells maintained their barrier function even after cryopreservation.

### Example 2: Verification of the effect of dilution with substrate for transplantation

It was confirmed in Example 1 that the spheroidized corneal endothelial substitute cells could be cryopreserved and maintained corneal endothelial function even after thawing. However, a cryopreservation solution is used for cryopreservation. Since the cryopreservation solution contains DMSO as a cryoprotective agent, washing is required before administration into the anterior chamber. In this Example, a method that does not require washing operation after thawing was investigated.

In order to verify whether the function of corneal endothelial substitute cells can be maintained without being affected by the cryopreservation solution, by diluting with a substrate for transplantation instead of washing, the barrier function was evaluated in the same manner as in Example 1 using the ability to form tight junction as an index. As a result, it was found that corneal endothelial substitute cells could be obtained while maintaining corneal endothelial function, by diluting the cells five times or more with a substrate for transplantation. Furthermore, animal tests are conducted to confirm that it can be safely administered into the anterior chamber.

### Experimental Example 1: Spike test (preliminary test to determine the amount of undifferentiated cells that may remain)

### (Method)

In order to determine the tolerable range of the proportion of undifferentiated cells that may remain in the therapeutic composition containing the corneal endothelial substitute cells of the present invention (also referred to as "undifferentiated cell residual rate"), the iPS cell spike test was conducted by the following method.
Method: Human fibroblasts were mixed with undifferentiated iPS cell line Ff-I01s04 (number of passages P=20) in the following numbers. iPS cell medium AK03N was mixed with a medium containing 10 µM of Y27632 (ROCK inhibitor), and this suspension was mixed with Matrigel at 1:1 and transplanted into the anterior chamber of nude rat eye at 1×10⁶ cells/rat. The tumorigenicity of the mixed undifferentiated iPS cells was amplified by transplanting them together with human fibroblasts as vegetative cells, an iPS cell medium, and Matrigel. As a result, a threshold value that is stricter and has a higher level of safety was determined.

### Number of undifferentiated Ff-I01s04 cells transplanted (spike rate)

1×10⁶ cells (100%)
1×10⁴ cells (1%)
1×10³ cells (0.1%)
1×10² cells (0.01%)

### (Judgment criteria)

### During the observation period;

When both of the following two items were met, it was determined that the rat "had macroscopic tumorigenicity", and the individual was euthanized, the transplanted eye was removed, and a definitive diagnosis was made through histopathological diagnosis.
i. The transplanted eye was swollen with clear left and right differences.
ii. The anterior chamber of the transplanted eye is filled with solid tissue.

### After the observation period;

After all individuals were euthanized, the transplanted eyes were removed and histopathological diagnosis was performed using HE staining.

### (Results)

Based on criteria i and ii, those whose teratoma formation in the anterior chamber was confirmed by histopathological diagnosis were considered "dead". A typical histological photograph of teratoma according to the spike rate is shown in Fig. 4, and a Kaplan-Meier curve is shown in Fig. 5.

The Ff-I01s04 undifferentiated iPS cell spike rate 100% (i.e., undifferentiated iPS cells only) group had a survival rate of 0% by 9 weeks. The undifferentiated iPS cell spike rate 1% group had a survival rate of 16.7% up to 16 weeks.

On the other hand, in the spike rate 0.1% and 0.01% groups, no teratoma formation was confirmed even in the final pathological diagnosis, and the survival rate was 100%.

### Example 3: Tumorigenicity test of iPS cell-derived corneal endothelial substitute cells in nude rats after single administration into the anterior chamber

### 1. Evaluation method of undifferentiated cell residual rate

The undifferentiated cell residual rate of iPS cell-derived corneal endothelial substitute cells (CECSi cells) used for tumorigenicity test in nude rats after single administration into the anterior chamber was evaluated according to a previous report (Stem Cell Res. 2021 Aug; 55:102497.).

In short, it is as follows.

Undifferentiated iPS cell lines (Ff-I01s04 and QHJI01s04) were mixed (spiked) with B4G12 cells at several concentrations and the expression level of OCT4 was analyzed. Using this data as a measure, the undifferentiated iPSC population remaining in each clone was quantified (Fig. 3). The expression level of OCT4 in CECSi cells was measured by qPCR (day 11). Using the spike test scale, it was confirmed that the remaining undifferentiated cells in the Ff-I01s04- or QHJI01s04-derived CECSi cells was not more than 0.1% (Fig. 3).

### 2. Tumorigenicity test

A therapeutic composition containing iPS cell-derived corneal endothelial substitute cells (undifferentiated cell residual rate not more than 0.1%) were administered (2.5×10⁴ cells/5 µL/eye) once into the anterior chamber (right eye) of nude rats (F344/NJcl-rnu/rnu; 4-week-old; CLEA Japan, Inc.) to examine tumorigenicity.
substrate for transplantation: DMEM/F12, ITS supplement (1×), IGF1 (10 ng/mL), Y27632 (100 pM)

The composition of the test group is as follows.

**[Table 1]**

| test substance group 1 | | | | | | | |
|---|---|---|---|---|---|---|---|
| group | test substance | dose (cells/ eye) | administered volume (µL/eye) | concentration (cells/mL) | autopsy timing | animal number (animal No.) | |
| | | | | | | male | female |
| 1 | CLS001 | 2.5×10⁴ | 5 | 5×10⁶ | 26w* | 13 | 13 |

| | | | | | | | |
|---|---|---|---|---|---|---|---|
| *: When the presence of cell aggregate is not confirmed in the anterior chamber on completion of the observation for 16 weeks after administration, the rats are autopsied. | | | | | | | |

Using a slit lamp, the anterior ocular segment and intermediate translucent body were examined and a photograph of the anterior ocular segment (right eye) was taken.

### <Criteria for determining tumorigenicity>

i. The right eye was swollen with clear left and right differences.
ii. The anterior chamber of the right eye is filled with solid tissue.

The above-mentioned therapeutic composition (undifferentiated cell residual rate: not more than 0.1%) was transplanted into the anterior chamber of nude rats, and 26 rats were followed up for up to 26 weeks.

From 12 weeks after transplantation, when cells were not observed in the anterior chamber for not less than 4 weeks in anterior ocular segment photographs, it was determined that the cells had disappeared. When cells were determined to have disappeared after 16 weeks after transplantation (Fig. 6a), autopsy was performed the next day of the determination of disappearance.

Animals with residual cells in the anterior chamber were observed until 26 weeks, and autopsy was performed the next day of the end of the observation period. As shown in Figs. 6b to 6e, there was no change in the size of the cell aggregate in the anterior chamber during progress, and no tumorigenicity was observed.

### Example 4: Verification of the size of spheroidized corneal endothelial substitute cells

### 1. iPS cell expansion culture (thawing)

Y-27632 (wako/039-24591) was added to iPS cell medium AK03N (Ajinomoto Co., Inc.) at a concentration of 10 µM (hereinafter referred to as AK03N+Y medium).

T12.5 flask (Corning Incorporated/353107) was coated with iMatrix511 (Matrixome/892005) at a concentration of 6 µg/mL. iPS cells (FF-I01s04 strain) were suspended in AK03N+Y medium at a cell concentration of 8.0×10³ cells/cm², and the resulting cell suspension was seeded into the T12.5 flask. Culture was started in a CO₂ incubator at 37°C, and the medium was replaced the next day with AK03N medium not containing Y-27632. After a gap of 2 days, medium exchange was performed for 2 consecutive days, and passage was performed on the 6th day of culture.

A T25 flask (SUMITOMO BAKELITE CO., LTD./MS-23050) was coated with iMatrix511 (Matrixome/892005) at a concentration of 6 µg/mL. iPS cells were suspended in AK03N+Y medium at a cell concentration of 2.2×10³ cells/cm², and the resulting cell suspension was seeded into the T25 flask. Culture was started in a CO₂ incubator at 37°C, and the medium was replaced the next day with AK03N medium not containing Y-27632. After a gap of 2 days, medium exchange was performed for 2 consecutive days, and passage was performed on the 6th day of culture.

### 2. Preparation of phenol red-free corneal endothelial substitute cell induction medium

DMEM/F12 nonphenol red (Thermo Fisher Scientific, Inc./11039-021) was used as the basal medium. To the basal medium (500 mL) were added, as additives, ITS-Supplement (Ventria/777ITS091), IGF1 (OrphanPacific, Inc.; Somazone for injection/858100075, 1 mg/mL), LIF (FUJIFILM Wako Pure Chemical Corporation/125-06661), IL-6 (Miltenyi Biotec/170-076-161), IL-11 (Pepro tech/AF-200-11), TNFα (Miltenyi Biotec/170-076-178), and water-soluble HYDROCORTONE Injection (Nichi-Iko Pharmaceutical Co., Ltd./620002208).

**[Table 2]**

| reagent name | amount added | final concentration |
|---|---|---|
| LIF | 200 µL | 10 ng/mL |
| IL-11 | 250 µL | 5 ng/mL |
| IL-6 | 250 µL | 10 ng/mL |
| TNFα | 100 µL | 10 ng/mL |
| ITS-Supplement | 5 mL | 10 mL/L |
| IGF1 | 5 µL | 10 ng/mL |
| HYDROCORTONE | 20 µL | 200 nM |

### 3. Verification of size

Using the corneal endothelial substitute cell induction medium described in Table 2 and by a method similar to that in Patent Literature 3, CECSi cells were induced from iPS cells. On the 14th day after induction, the cells were collected using Accutase (Innovative Cell Technologies, Inc. #AT104-100ML), suspended in cell preservation solution BAMBANKER (registered trade mark) hRM (NIPPON Genetics Co, Ltd. #CS-07-001) at a cell concentration of 1×10⁷ cells/mL, and cryopreserved in a deep freezer at -80°C. The cryopreserved corneal endothelial substitute cell was thawed, immediately thereafter suspended in a spheroid-forming medium described below, seeded on a large spheroid-forming bag (TOYO SEIKAN CO. LTD., WellBag) at a cell count of 1.2 × 10⁸ cells, and spheroid-forming medium was added to finally about 400 mL. This was cultured for one day in an incubator at 37°C to form spheroids (cell aggregates) of CECSi cells. CECSi cell spheroids were washed and concentrated by a CTS Rotea Counterflow Centrifugation System (Thermo Fisher Scientific, Inc.). A concentrated portion was sampled, and the spheroids were enzyme-treated with Accumax (Innovative Cell Technologies, Inc. #AM105) and the number of cells was counted. Using this measurement result, the spheroids were suspended in the cell preservation solution BunBunker (registered trademark) hRM so that the cell number was 7.5×10⁶ cells/mL, 40 µL each was dispensed, and cryopreserved at -70°C or below. After thawing, the cells were diluted 5 times with DMEM/F12 (Thermo Fisher Scientific, Inc./11330-032), which is used as a basal medium for substrate for transplantation (final cell concentration: 1.5×10⁶ cells/mL), and iSpect DIA-10 (Shimadzu Corporation) was used to measure the number of spheroids with an area equivalent diameter (the area of a particle and the diameter of a circle having the same area as the particle) of 30 um or more. The results are shown in Fig. 7. As a result of the analysis, the spheroid diameter (area equivalent diameter of spheroid) was about 30 to 150 µm, and not less than 90% thereof was cell populations in the range of about 30 to 90 um. When the spheroid diameter is within this range, the engraftment effect of the therapeutic composition can be maintained.

### Example 5: Examination of DMSO concentration of spheroidized corneal endothelial substitute cells

Using the corneal endothelial substitute cell induction medium described in Table 2 and by a method similar to that in Patent Literature 3, CECSi cells were induced from iPS cells. On the 14th day after induction, the cells were collected using Accutase (Innovative Cell Technologies, Inc. #AT104-100ML), suspended in cell preservation solution BAMBANKER (registered trade mark) hRM (NIPPON Genetics Co, Ltd. #CS-07-001, containing 10% DMSO) at a cell concentration of 1×10⁷ cells/mL, and cryopreserved in a deep freezer at -80°C. The cryopreserved corneal endothelial substitute cell was thawed, immediately thereafter suspended in a spheroid-forming medium described below, and seeded on a large spheroid-forming bag (TOYO SEIKAN CO. LTD., WellBag) at a cell count of 1.2 × 10⁸ cells/bag. This was cultured for one day in an incubator at 37°C to form spheroids (cell aggregates) of CECSi cells. Spheroidized CECSi cells were collected from the spheroid-forming bag and washed. Then, using cell preservation solution BAMBANKER (registered trademark) hRM (NIPPON Genetics Co, Ltd. #CS-07-001, containing 10% DMSO), the cells were prepared to a cell concentration of 7.5×10⁶ cells/mL. The prepared spheroid suspension was dispensed into a primary container at a cell concentration of 3×10⁵ cells/40 µL and cryopreserved at not more than -70°C.

### <Preparation of spheroid-forming medium>

As the basal medium, DMEM/F12 (Thermo Fisher Scientific, Inc./11330-032) or DMEM/F12 no phenol red (Thermo Fisher Scientific, Inc./21041-025) was used. ITS-Supplement (Ventria/777ITS091), Y-27632 (FUJIFILM Wako Pure Chemical Corporation/039-24591), and IGF1 (OrphanPacific, Inc.; Somazone for injection/858100075, 1 mg/mL) were added as additives to 40 mL of the basal medium.

**[Table 3]**

| reagent name | amount added | final concentration |
|---|---|---|
| ITS-Supplement | 400 µL | 10 mL/L |
| Y-27632 | 40 µL | 100 µM |
| IGF1 | 2 µL | 50 ng/mL |

Spheroidized CECSi cells after cryopreservation were seeded into 48-well plates (iMatrix511MG coated) using substrate for transplantation at various DMSO concentrations (100%, 50%, 25%, 10%, 5%, 2%, 1%, 0%), and the cells were allowed to adhere to the plates. After 24 hr, the medium was removed from the spheroids on the wells and the spheroids were fixed with ice-cooled 4% para-formaldehyde. After washing the wells twice with PBS, blocking solution (PBST containing 10% Normal Donkey Serum (10% NDS/PBST)) was added and blocking was performed for 30 min. Thereafter, a primary antibody reaction was performed at room temperature for 1 hr using the following antibody amounts.
Rabbit anti human ZO-1; Invitrogen, 40-2200, 1:500, 0.5 µL/well Mouse anti human Na,K-ATPaseα1; Novus, NB300-146, 1:500, 0.5 µL/well
blocking solution (10% NDS/PBST), 0.25 mL/well

Then, the well was washed twice with PBS, and a secondary antibody reaction was performed at room temperature for 1 hr using the following antibody amounts.
Donkey anti-rabbit Cy-3, Jackson, 711-165-152, 1:200, 1.25 µL/well
Donkey anti-mouse Alexa flour 488, Invitrogen, A21202, 1.67 µL/well
DAPI, 1:1000, 0.25 µL/well blocking solution (10% NDS/PBST), 0.25 mL/well

Then, after washing twice with PBS, a mounting agent was added and observed with a fluorescence microscope. It was confirmed that when the DMSO concentration at the time of transplantation is not more than 5%, the engraftment ability and the tight junction formation ability are present.

### Experimental Example 2: Screening of CECSi cell surface marker using FCM

Screening was performed using the Human Cell Surface Marker Screening Panel (Lyoplate) and the expression of cell surface antigen in CECSi cells was analyzed with a flow cytometer (BD Biosciences, BD LSRFortessa^{™} X-20). Data analysis was performed using BD FACSDiva software ver. 8.0.1 (Becton, Dickinson and Company).

Three lots of CECSi cells were prepared (Lot.2020F1, Lot.2020G01, Lot.2020I24), each was subjected to a predetermined antibody reaction, and immediately before FCM measurement, an appropriate amount of 7-AAD (BD Biosciences #559925) was added to stain the cells. Strong positive (⊙) indicates that AF647 (Alexa Fluor 647) was detected in not less than 80%, positive (○) indicates that AF647 was detected in not less than 10% and less than 80%, and weak positive indicates that AF647 was detected in not less than 5% and less than 10%.

As a result, CD24 was found as a cell surface marker characteristic of CECSi cells.

**[Table 4]**

| marker | Lot.2020F1 | Lot.2020G01 | Lot.2020I24 |
|---|---|---|---|
| Buffer | - | - | - |
| CD24 | ⊙ | ⊙ | ⊙ |

### Experimental Example 3: Comparison of B2M expression level

DNA microarray analysis using real human corneal endothelial cells collected from overseas donor research corneas, iPS cells (Ff-I01s04), and CECSi cells was performed using DNA microarray analysis (SurePrint G3 Human GE microarray 8x60K Ver. 3.0) manufactured by Agilent Technologies, Inc. to comprehensively determine the gene profile. Among the results, the expression level of beta-2 microglobulin (B2M), which is one of the factors constituting Human Leukocyte Antigen (HLA), was compared. It was shown that the B2M expression levels in iPS cells and CECSi cells were relatively low compared to human corneal endothelial cells (Fig. 3). Therefore, during allogenic transplantation, CECSi cells have characteristic that they are less likely to cause rejection than human corneal endothelial cells.

### Example 6: Verification of the effect of phenol red on spheroid formation

The cells were seeded at a cell count of 2.5×10⁶ cells/bag on a medium-size spheroid-forming bag (TOYO SEIKAN CO. LTD., WellBag, volume 20 mL) filled with a spheroid-forming medium. By culturing same in an incubator at 37°C for one day, spheroids of CECSi cells were formed. The CECSi cell spheroids were collected in a 15 mL centrifuge tube (SUMITOMO BAKELITE CO., LTD. #MS-90150), and after recovery by centrifugation (conditions: 200 g, 5 min, 25°C), microscopic observation was performed (Fig. 9) and total viable cell numbers were measured (Table 5). As a result, both the number of spheroids produced and the total viable cells were higher when spheroids were formed using a spheroid-forming medium using DMEM/F12 no phenol red as the basal medium. Spheroids can be formed more easily by using a medium that does not contain phenol red or by adding a step of removing phenol red.

**[Table 5]**

| | number of total viable cells (DMEM/F12 no phenol red) | number of total viable cells (DMEM/F12) |
|---|---|---|
| CECSi (Lot.1) | 2.61×10⁵ cells | 1.20×10⁵ cells |
| CECSi (Lot.2) | 0.73×10⁵ cells | 0.43×10⁵ cells |

### [Industrial Applicability]

According to the present invention, a therapeutic composition, particularly a composition for transplantation therapy, which can be cryopreserved, does not cause damage to cells due to thawing, and can be administered to a patient as is without undergoing additional processes such as washing or centrifugation of cells after thawing can be provided. The therapeutic composition of the present invention is useful for transplanting corneal endothelial substitute cell in bullous keratopathy, which accounts for about 50% of corneal transplantation applicable cases worldwide.

This application is based on a US provisional patent application No. 63/219,086 filed in the US (filing date: July 7, 2021) and a US provisional patent application No. 63/320,846 filed in the US (filing date: March 17, 2022), the contents of which are incorporated in full herein.

## Claims

1. A therapeutic composition comprising a corneal endothelial substitute cell having a CD24-positive phenotype, and further a phenolphthalein derivative when desired.

2. The composition according to claim 1, further comprising a cryoprotective agent.

3. The composition according to claim 2, wherein the cryoprotective agent is dimethyl sulfoxide (DMSO).

4. The composition according to claim 3, wherein a concentration of the DMSO in the composition is 1 to 10%.

5. The composition according to any one of claims 1 to 4, wherein the phenolphthalein derivative is selected from the group consisting of phenolsulfonphthalein (phenol red), phenolphthalein, bromophenol blue, isovaleryl phenolphthalein, acetylphenolphthalein, phenolphthalein dibutyrate, phenolphthalein diphosphate, phenolphthalein disulphate, phenolphthalein glucuronic acid, phenolphthalein mono-β-glucoside uronic acid, phenolphthalein mono-β-glucuronic acid, phenolphthalein mono-phosphate, and cresol red.

6. The composition according to any one of claims 1 to 4, wherein the phenolphthalein derivative is phenol red.

7. The composition according to claim 6, wherein a concentration of the phenol red in the composition is 1 to 20 mg/L.

8. The composition according to any one of claims 1 to 7, wherein a concentration of the cell in the composition is 0.5 to 10×10⁶ cells/mL.

9. The composition according to any one of claims 1 to 8, which is in a dosage form of a spheroid cell suspension.

10. The composition according to claim 9, wherein a cell population in the composition comprises spheroids having an area equivalent diameter of not less than 30 µm, and not less than 50% of the spheroids have an area equivalent diameter of 30 to 90 um.

11. The composition according to any one of claims 1 to 10, wherein the corneal endothelial substitute cell is derived from a stem cell.

12. The composition according to claim 11, wherein the stem cell is a pluripotent stem cell.

13. The composition according to claim 12, wherein the pluripotent stem cell is derived from an iPS cell (including a cell imparted with an additional function by gene editing or gene transfer).

14. The composition according to claim 13, wherein a B2M expression level is lower than that of a human corneal endothelial cell or a cultured endothelial cell.

15. The composition according to any one of claims 1 to 14, which is for transplantation.

16. The composition according to claim 15, which is for transplantation into the anterior chamber.

17. A method for producing a therapeutic composition, comprising adding a substrate for transplantation comprising a phenolphthalein derivative to a cell suspension or cell spheroid suspension of corneal endothelial substitute cells which is frozen at not more than -20°C, in the presence of a cryoprotective agent.

18. The method according to claim 17, wherein the corneal endothelial substitute cell has a CD24-positive phenotype.

19. A therapeutic composition comprising a corneal endothelial substitute cell derived from a stem cell, and having an undifferentiated cell residual rate of less than 1%.

20. The composition according to claim 19, wherein the corneal endothelial substitute cell has a CD24-positive phenotype.

21. The composition according to claim 19 or 20, which has an undifferentiated cell residual rate of not more than 0.1%.

22. The composition according to any one of claims 19 to 21, wherein the stem cell is a pluripotent stem cell.

23. The composition according to claim 22, wherein the pluripotent stem cell is derived from an iPS cell (including a cell imparted with an additional function by gene editing or gene transfer).

24. The composition according to any one of claims 19 to 23, wherein a B2M expression level is lower than that of a human corneal endothelial cell or a cultured endothelial cell.

25. The composition according to any one of claims 19 to 24, wherein the undifferentiated cell residual rate is evaluated by expression profile of an undifferentiated cell marker.

26. The composition according to any one of claims 19 to 25, which is for transplantation.

27. The composition according to claim 26, which is for transplantation into the anterior chamber.
